# EUROPÄISCHE PATENTSCHRIFT

(11) **EP 0 919 182 B1**
(45) Veröffentlichungstag und Bekanntmachung des Hinweises auf die Patenterteilung: **10.08.2005**
(21) Anmeldenummer: 98122480.1
(22) Anmeldetag: 26.11.1998
(51) Int. Cl.: A61B 5/0215

(54) **Druckmesswandleranordnung, insbesondere zur invasiven Blutdruckmessung**
Pressure measuring arrangement, particularly for the invasive blood pressure measurement
Disposition de mesure de pression, notamment pour la mesure invasive de la pression sanguine

(30) Priorität: 28.11.1997 DE 19752856; 06.11.1998 DE 19851276
(43) Veröffentlichungstag der Anmeldung: 02.06.1999
(73) Patentinhaber: Smiths Medical Deutschland GmbH, 85614 Kirchseeon (DE)
(72) Erfinder: Müller, Daniel, 85614 Kirchseeon (DE)
(74) Vertreter: von Bülow, Tam, Dr.

(56) Entgegenhaltungen:
- EP-A- 0 672 381
- WO-A-97/39679

## Beschreibung

Die Erfindung bezieht sich auf eine Druckmeßwandler-Anordnung gemäß dem oberbegriff des Patentanspruches 1 und gemäß dem Oberbegriff des Patentanspruches 11.

Eine derartige Druckmeßwandler-Anordnung ist aus der EP 0 672 381 A1 bzw. der EP 0 447 457 B1 bekannt. Bei der EP 0 672 381 sind mehrere einmal verwendbare Druckmeßwandler an einem gemeinsamen Halteelement fixierbar und über Kontakte und Gegenkontakte mit einer einzigen gemeinsamen Signalübertragungseinrichtung für alle Druckmeßwandler verbindbar, wobei die Signalübertragungseinrichtung mit einem Monitor verbindbar ist.

Die EP 0 447 457 B zeigt einen Druckmeßwandler mit Druckdom und Membran.

Die EP 0 124 308 A2 zeigt eine Druckmeßwandler-Anordnung mit drei Komponenten, nämlich einem einmal verwendbaren Druckmeßwandler, einem Halteelement und einem Verbindungskabel zu einem Monitor. In einem Gehäuse des Druckmeßwandlers ist das eigentliche Druckmeßelement gekapselt angeordnet, das mit einer Kammer, die mit dem zu messenden Fluiddruck beaufschlagbar ist, in Verbindung steht. Das Druckmeßelement hat elektrische Steckkontakte, die in Gegenkontakte des Halteelementes einschiebbar sind. Das Halteelement hat ebenfalls ein Gehäuse, das so geformt ist, daß es das Druckmeßwandlergehäuse aufnimmt und hält. Die Gegenkontakte am Halteelementgehäuse sind mit dem Kabel verbunden. Nach Gebrauch kann das Druckmeßwandlergehäuse von Hand aus dem Halteelement entfernt und durch ein neues Druckmeßwandlergehäuse ausgetauscht werden.

Eine ähnliche Druckmeßwandler-Anordnung ist aus der DE 44 00 941 bekannt, die ebenfalls aus drei Komponenten besteht, nämlich einem Druckmeßwandler, einem Kabel mit Stecker und einem Halteelement zur Fixierung des Druckmeßwandlers und des Steckers.

Der Druckmeßwandler weist ein Gehäuse mit zwei Kammern, einem Flüssigkeitskanal und zwei Flüssigkeitsanschlüssen auf. Der eine Anschluß ist mit dem Gefäßsystem eines Patienten und der andere mit einer Infusionsflasche verbunden, was eine Infusion von z. B. physiologischer Kochsalzlösung gestattet um Blutkoagulationen im Leitungssystem zu verhindern. In einer zwischen den beiden Anschlüssen liegenden Gehäusekammer ist ein Druckmeßelement angeordnet, das auf einem Substrat fixiert ist und das mit dem Flüssigkeitskanal in Druckverbindung steht. Ferner sind auf dem Substrat Leiterbahnen vorgesehen, die mit dem Druckmeßelement und weiteren elektrischen Komponenten einer Meßschaltung verbunden sind. Das Substrat und die Leiterbahnen sind bis in das Innere der zweiten Gehäusekammer geführt. Dort bilden die Enden der Leiterbahnen Kontakte, die über eine Öffnung in der Gehäusewand zugänglich sind. Der Stecker ist komplementär bezüglich der Gehäuseöffnung und den Kontakten gestaltet und weist den Kontakten zugeordnete Gegenkontakte auf, die mit Adern des Kabels verbunden sind. Das Halteelement weist an ihrer Oberseite Halteschienen zum Einschieben des Druckmeßwandlers auf.

Bei Blutdruckmessungen ist der Druckmeßwandler an dem Halteelement fixiert und der Stecker ist so eingeschoben, daß sich die Kontakte und die Gegenkontakte berühren. Ein im Druckmeßwandler anliegender Blutdruck wird vom Druckmeßelement abgenommen und in ein elektrisches Signal umgewandelt, das über das Kabel einem Monitor zugeführt wird. Bei Operationen oder Patienten auf Intensivstationen werden oft mehrere Drücke gleichzeitig gemessen, wozu mehrere Druckmeßwandler eingesetzt werden. Dementsprechend sind die bekannten Halteelemente modular aufgebaut, so daß mehrere Halteelemente mit je einem Druckmeßwandler aneinander gesteckt werden können. Von diesen Halteelementen führen dann getrennte Kabel, je eines pro Druckmeßwandler, zum Monitor. Die Anzahl von Kabeln und Schläuchen macht dann die ganze Meßanordnung recht unübersichtlich, birgt die Gefahr von Vertauschungen beim Auswechseln der Druckmeßwandler in sich und auch, daß das Pflegepersonal über die Kabel stolpert.

Weiter werden die Druckmeßwandler bei einem Patienten in regelmäßigen Zeitabständen ausgetauscht, entsorgt und durch neue ersetzt, während die Halteelemente und die Kabel wieder verwendet werden (vgl. DE 43 17 985 A1). Beim Austausch der Druckmeßwandler müssen die Schläuche abgenommen und an die neuen Druckmeßwandler angeschlossen werden. Bei diesem Vorgang kann unbeabsichtigt Blut oder Infusionsflüssigkeit auf den neuen Druckmeßwandler oder die Halteelemente verspritzt werden.

Druckmeßwandler der hier beschriebenen Art haben üblicherweise eine Wheaton'sche Brückenschaltung, deren einer Brückenzweig einen drucksensitiven Widerstand aufweist. Zur Funktionsüberprüfung ist es üblich, diesen Brückenzweig durch einen definierten Widerstand zu überbrücken. Am Monitor wird dann üblicherweise ein Prüfwert entsprechend 100 mm/Hg angezeigt. Das Zuschalten dieses Widerstandes erfolgt durch einen Taster, der üblicherweise am Monitor, am Druckmeßwandler selbst (DE 44 00 941) oder zusammen mit einem Shunt-Widerstand auf dem Kabel (EP 0 366 651 C2 und US 4,603,574) angeordnet ist. Im Falle der Anordnung am Monitor oder auf dem Kabel ist dieser Taster wiederverwendbar, auch wenn der Druckmeßwandler nach Gebrauch entsorgt wird.

Aus der DE 295 06 589 U1 ist eine Druckmeßwandler-Anordnung bekannt, die mehrere sogenannte "Druckdome" aufweist, die jeweils mit dem Patienten und mit der Infusionsflasche verbunden sind und die nach einmaligem Gebrauch entsorgt werden. Die Druckdome sind auf wiederverwendbaren Halteelementen fixiert, die modulartig aneinandergesteckt sind. Jedes Halteelement weist ein wiederverwendbares Druckmeßelement mit einer Druckübertragungsmembran auf, die mit einer Druckübertragungsmembran zusammenwirkt, die außen am zugeordneten Druckdom vorgesehen ist. Auch hier führt von jedem Druckmeßelement ein eigenes Kabel weg, was zu den oben genannten Problemen führt.

Aufgabe der Erfindung ist es, die Druckmeßwandler-Anordnung der eingangs genannten Art dahingehend zu verbessern, daß die Signalübertragungseinrichtung auswechselbar ist.

Diese Aufgabe wird durch die in den Patentansprüchen 1 und 11 angegebenen Merkmale gelöst. Vorteilhafte Ausgestaltungen und Weiterbildungen der Erfindung sind den Unteransprüchen zu entnehmen.

Grundidee der Erfindung ist es, die Signalübertragungseinrichtung, insbesondere wenn es sich um ein Kabel handelt, von Hand von dem Halteelement lösbar auszubilden. Damit können das Halteelement und/oder das Kabel bei Bedarf einzeln ausgewechselt werden. In der Praxis hat sich nämlich herausgestellt, daß die Kabel stark strapaziert werden und öfters ausgetauscht werden müssen. Zusätzlich sind die monitorseitigen Stecker des Kabels ebenfalls einzeln von Hand austauschbar. Diese an dem Monitor anzuschließenden Stecker haben in der Praxis nur einen sehr geringen Verschleiß und sind darüber hinaus extrem teuer und müssen nach dieser Ausgestaltung der Erfindung nicht mehr ausgetauscht werden.

Ein weiterer Vorteil der Erfindung liegt darin, daß das Halteelement gleichzeitig die Funktion der sonst üblichen Halteplatte (vgl. DE 43 17 985 A1) übernimmt. Auch bei der EP 0 124 308 wird üblicherweise das dort verwendete Halteelement noch zusätzlich an einer Halteplatte befestigt, die ihrerseits üblicherweise an einem Infusionsständer angebracht ist. Damit mußte beim Stand der Technik in zwei Arbeitsschritten zunächst der Druckmeßwandler am Halteelement aufgesteckt und dann diese Einheit an der Halteplatte befestigt werden. Bei der Erfindung dagegen erfolgt die Herstellung der elektrischen oder druckübertragenen Verbindung zwischen dem Druckmeßelement bzw. dem Druckdom und dem Halteelement und die Fixierung am Infusionsständer in einem Arbeitsgang. Ein weiterer Vorteil liegt darin, daß nur noch eine einzige Signalübertragungseinrichtung und insbesondere ein einziges Kabel zum Monitor führt. Verwechslung oder Vertauschung der Kabel sind damit ausgeschlossen.

Bei Verwendung eines Kabels muß im übrigen auch nicht mehr zu jedem Gegenkontakt eine eigene Ader des Kabels geführt werden. Gegenkontakte für Spannungsversorgung und Masse können je über eine einzige Ader des Kabels erfolgen, wobei dann lediglich innerhalb des Halteelementes eine entsprechende Verdrahtung bzw. eine gedruckte Leiterplatte vorhanden ist. Damit benötigt das Kabel weniger Adern und kann entsprechend dünner sein, wodurch nebenbei auch noch die Kosten reduziert werden. Diese Vorteile gelten selbstverständlich auch für Druckmeßwandler-Anordnungen mit den einmal verwendbaren Druckdomen.

Ein Folgeproblem der Druckmeßwandler-Anordnung nach der Erfindung kann darin liegen, daß beim Lösen von Zuleitungsschläuchen auch Flüssigkeit, wie z. B. physiologische Kochsalzlösung, auf das Halteelement und insbesondere die Kontakte oder auch auf den neuen Druckmeßwandler und dessen Kontakte gelangen kann. Physiologische Kochsalzlösung ist ein relativ guter elektrischer Leiter, so daß durch einen sich zwischen benachbarten Kontakten ausbildenden Flüssigkeitsfilm ein Nebenschlußwiderstand entsteht, der das Meßergebnis verfälschen kann. Zur Lösung dieses Folgeproblems schlägt die Erfindung prinzipiell vor, die Kontakte und/oder Gegenkontakte so anzuordnen und/oder auszubilden, daß ein die Kontakte überbrückender Flüssigkeitsfilm sich in der Praxis nicht ausbilden kann oder bei der Herstellung der elektrischen Verbindung zwangsweise unterbrochen oder abgewischt wird. Hierzu können beispielsweise rings um die Kontakte und/oder die Gegenkontakte Dichtorgane angeordnet sein, die einen Flüssigkeitsfilm verdrängen. Auch kann vorgesehen sein, daß Kontakte und/oder Gegenkontakte erst bei der mechanischen Verbindung des Druckmeßwandlers mit dem Halteelement die elastischen Dichtungen durchstoßen und vorher vor Flüssigkeit geschützt sind. Auch für die Gestaltung der Kontakte und der Gegenkontakte bestehen vielfältige Möglichkeiten, wie z. B. Berührkontakte, Klemmkontakte, Stecker-Buchsenkombinationen, federvorgespannte Kontaktstifte, Schneidklemmkontakte oder sonstige elektrische Verbindungsmittel.

Schließlich ist nach einer Weiterbildung der Erfindung vorgesehen, daß die eingangs genannte Prüftaste einschließlich deren elektrische Kontakte am bzw. im Halteelement angeordnet sind.

Im folgenden wird die Erfindung anhand von Ausführungsbeispielen im Zusammenhang mit der Zeichnung ausführlicher erläutert. Es zeigen:
- Fig. 1: eine schematische Darstellung einer Druckmeßwandler-Anordnung gemäß der Erfindung mit einem Halteelement zur Fixierung und zum Anschluß mehrerer Druckmeßwandler;
- Fig. 2: eine Ansicht eines geöffneten Halteelementes, in der die Verdrahtung der Gegenkontakte zu sehen ist;
- Fig. 3: ein Ausführungsbeispiel einer Druckmeßwandler-Anordnung mit federbelasteten Kontaktstiften am Halteelement;
- Fig. 4: ein Ausführungsbeispiel mit Klemm- und Stiftkontakten;
- Fig. 5a, 5b und 5c: ein Ausführungsbeispiel mit parallel zur Halteelementebene angeordneten Stiftkontakten;
- Fig. 6: ein Ausführungsbeispiel mit Berührkontakten;
- Fig. 7: ein Ausführungsbeispiel mit feuchtigkeitsgeschützten Druckmeßwandler-Kontakten;
- Fig. 8: ein Ausführungsbeispiel mit versenkbaren Halteplattenkontakten;
- Fig. 9a: ein Ausführungsbeispiel, bei dem mehrere Druckdome einzeln auf dem Halteelement befestigbar sind;
- Fig. 9b: ein Ausführungsbeispiel, bei dem mehrere miteinander verbundene Druckdome als eine Einheit am Halteelement befestigbar sind;
- Fig. 10a: ein Ausführungsbeispiel ähnlich dem der Fig. 1, wobei das Kabel über eine Steckverbindung mit Monitorkabeln verbunden ist;
- Fig. 10b: ein Ausführungsbeispiel ähnlich dem der Fig. 10a, bei dem zusätzlich unmittelbar am Halteelement eine Steckverbindung für den Anschluß des Kabels vorgesehen ist. und
- Fig. 10c: ein Ausführungsbeispiel mit kabelloser Signalübertragung.

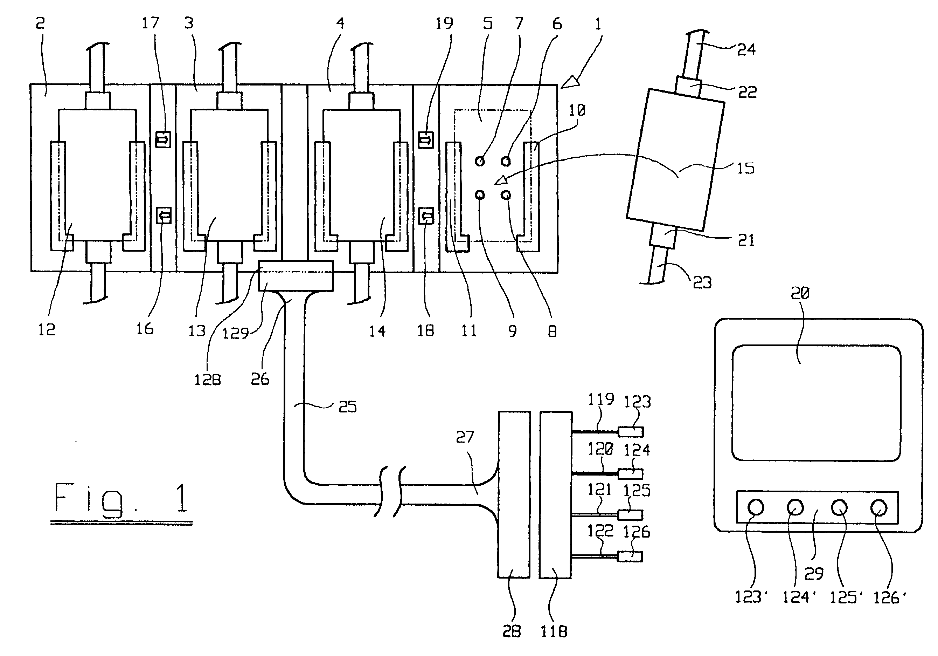

Fig. 1 zeigt eine schematische Darstellung einer Druckmeßwandler-Anordnung mit einem Halteelement 1, die vier Druckmeßplätze 2, 3, 4 und 5 aufweist. An jedem der Druckmeßplätze 2, 3, 4 und 5 sind je vier Halteplattenkontakte 6, 7, 8 und 9 vorgesehen, die hier unmittelbar an der gezeigten Außenfläche der Vorderseite des Halteelementes 1 angeordnet sind. Ferner sind an den Druckmeßplätzen 2, 3, 4 und 5 je zwei Rastelemente 10 und 11 zum lösbaren Fixieren von Druckmeßwandlern 12, 13, 14 und 15 vorgesehen. In der Darstellung der Fig. 1 sind die Druckmeßwandler 12, 13 und 14 auf den entsprechenden Druckmeßplätzen 2, 3 und 4 des Halteelementes 1 befestigt, während der Druckmeßwandler 15 abgenommen ist.

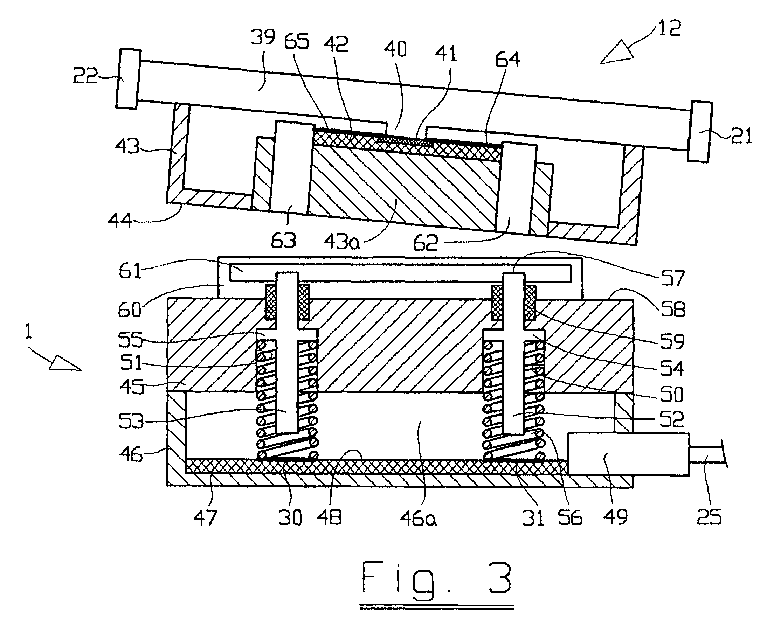

Jeder der hier nur schematisch dargestellten Druckmeßwandler 12, 13, 14 und 15 weist einen ersten Flüssigkeitsanschluß 21 und einen zweiten Anschluß 22 auf, mit denen jeweils Schläuche 23 bzw. 24 verbunden sind. Beispielsweise steht der Anschluß 21 über den Schlauch 23 mit einem Blutgefäß eines Patienten in Verbindung, während der andere Schlauch 24 mit einer Infusionsflasche verbunden sein kann. Im Innern der Druckmeßwandler 12, 13, 14 und 15 ist je ein Druckmeßelement (gezeigt in Fig. 3) enthalten, das bei am Halteelement 1 fixiertem Druckmeßwandler 12, 13, 14 bzw. 15 mit zugeordneten Halteplattenkontakten 6, 7, 8 und 9 elektrisch verbunden ist.

Jedem der Druckmeßwandler 12, 13, 14 und 15 ist eine auf dem Halteelement 1 vorgesehene Prüftaste 16, 17, 18 und 19 zugeordnet. Die Prüftasten 16, 17, 18 und 19 ermöglichen Funktionsprüfungen der zugeordneten Druckmeßwandler 12, 13, 14 bzw. 15. Hierzu sind elektrische Kontakte der Prüftasten 16, 17, 18 und 19 im Inneren des Halteelementes 1 mit zugeordneten Halteplattenkontakten verbunden, wobei durch Betätigen der Prüftasten ein Widerstand eines Brückenzweiges im zugeordneten Druckmeßwandler in definierter Weise verändert wird. Alternativ hierzu kann für alle Druckmeßwandler auch nur eine einzige Prüftaste vorgesehen sein, die gleichzeitig die Brückenzweige aller Druckmeßwandler in definierter Weise verändert.

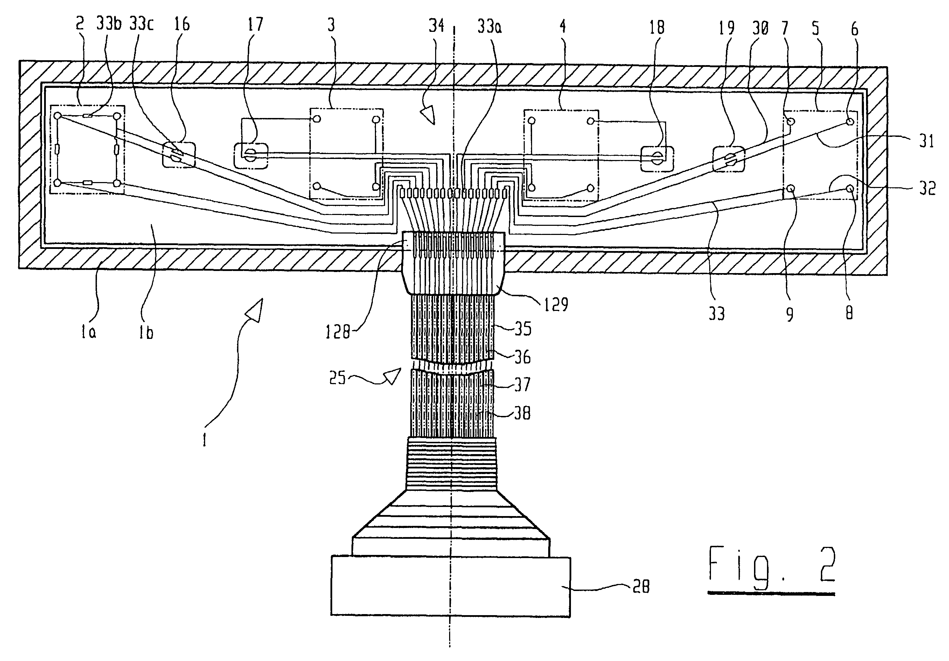

An dem Halteelement 1 ist ferner ein Kabel 25 angebracht, dessen Adern (Fig. 2) mit je einem zugeordneten Halteplattenkontakt elektrisch verbunden sind, wobei ein erstes Ende 26 des Kabels 25 mit dem Halteelement 1 verbunden ist. Am freien Ende 27 des Kabels 25 ist ein Steckerteil 28 vorgesehen. Das Steckerteil 28 kann so ausgeführt sein, daß es direkt in ein Buchsenteil 29 eines Monitors 20 einsteckbar. Auch am Halteelement-seitigen Ende des Kabels 26 kann ein Stecker 129 angebracht sein mit entsprechenden Gegenkontakten 128 am oder im Halteelement 1. Alternativ kann das Halteplatten-seitige Ende 26 des Kabels aber auch an einer Platine (Fig. 2) im Halteelement 1 angelötet sein.

Bei der in Fig. 1 dargestellten Variante hat das monitorseitige freie Ende 27 des Kabels 25 ein Steckerteil 28, das mit einem Gegenstecker 118 verbindbar ist, aus welchem pro Meßkanal einzelne, kurze Kabel 119, 120, 121 und 122 herausragen, an deren Ende jeweils ein Monitorstecker 123, 124, 125 bzw. 126 befestigt ist, die in zugeordnete Buchsen 123', 124', 125' bzw. 126' am Monitor 20 einsteckbar sind. Damit kann das Kabel 25 mit den beiden Steckern 129 und 28 ausgewechselt werden, ohne daß die teuren Monitorstecker 123-126 ausgewechselt werden müssen.

Fig. 2 zeigt eine teilweise geschnittene Vorderansicht eines geöffneten Halteelementes 1, mit einem Gehäuse 1a, in das eine bedruckte Leiterbahnplatte 1b eingesetzt ist, auf welcher die elektrischen Verbindungen der Halteplattenkontakte 6, 7, 8 und 9 mit dem Kabel 25 zu sehen sind. Entsprechend dem in der Fig. 1 gezeigten Halteelement 1 sind vier Druckmeßplätze 2, 3, 4 und 5 vorgesehen, wovon jeder vier Halteplattenkontaktstellen aufweist, die exemplarisch am Befestigungsplatz 5 mit den Bezugszeichen 6, 7, 8 und 9 bezeichnet sind. Jeder Halteplattenkontakt 6, 7, 8 und 9 ist mit je einer Leitung 30, 31, 32 bzw. 33 verbunden, die in einen Mittelbereich 34 des Halteelementes 1 geführt sind und dort mit zugeordneten Adern 35, 36, 37 bzw. 38 des Kabels 25 elektrisch verbunden sind. Auf der Leiterbahnplatte 1b sind zusätzlich diverse elektrische Widerstände 33a aufgedruckt, die hier in jeder Leitung als Vorwiderstand dargestellt sind und z.B. zur Kompensation von Temperaturfehlern dienen können und/oder als Spannungsteiler, um den eigentlichen Meßschaltkreis symmetrisch zur Versorgung zu legen. In ähnlicher Weise können an den einzelnen Meßplätzen zwischen den Halteplattenkontakten 6, 7, 8 und 9 jeweils Widerstände 33b aufgedruckt sein, die Teile der Brückenschaltung bzw. Abgleichwiderstände bilden. Auch sind auf der Leiterbahnplatte 1b im Bereich der Prüftasten 16-19 Schaltkontaktflächen 33c aufgedruckt, jeweils in elektrischer Verbindung mit der zugeordneten Leiterbahn, wobei die im hier nicht dargestellten Gehäusedeckel verschieblich geführte, ebenfalls nicht dargestellte Prüftaste an ihrer der Leiterbahnplatte 1b zugewandten Seite eine elektrisch leitfähige Beschichtung aufweist, die beim Niederdrücken die beiden Schaltkontaktflächen 33c kontaktiert und damit die zugehörigen Leitungen verbindet.

Die beiden Leitungen 30 und 31 dienen beispielsweise als Versorgungsleitungen für den zugeordneten Druckmeßwandler 12 (Fig. 1) und die anderen beiden Leitungen 32 und 33 als Datenleitungen zur Übertragung von Meßsignalen vom Druckmeßwandler 12 über den Stecker 28 zum daran angeschlossenen Monitor 20.

Jedem der Druckmeßplätze 2, 3, 4 und 5 ist je eine Prüftaste 16, 17, 18 und 19 zugeordnet, die hier nur schematisch dargestellt sind. Durch Betätigen der Prüftaste 19, die dem Steckplatz 5 zugeordnet ist, werden die beiden mit den Kontakten 6 und 7 verbundenen Leitungen 30 bzw. 31 überbrückt, was bei ordnungsgemäßer Funktion eines am Druckmeßplatz 5 fixierten Druckmeßwandlers 12 zu einem vordefinierten elektrischen Prüfsignal führt, das am Monitor 20 (Fig. 1) abgelesen werden kann. Tritt am Druckmeßwandler 12 oder an den elektrischen Kontakten 6, 7, 8 bzw. 9 eine Funktionsstörung auf, so weicht bei Betätigen der Prüftaste 19 das Prüfsignal von dem vordefinierten Wert ab.

Im dargestellten Ausführungsbeispiel ist das Kabel 25 mit einer Stecker-/Buchsenverbindung 128, 129 mit den einzelnen Leitungen, wie z.B. 30-33, verbunden. Das Buchsenelement ist dabei an der Leiterbahnplatte 1b und/oder am Gehäuse 1a befestigt. Alternativ hierzu kann das Kabel 25 auch direkt an den einzelnen Leitungen der Leiterbahnplatte angelötet sein.

Aus Fig. 2 ist zu erkennen, daß wesentliche Komponenten, wie Widerstände 33a, 33b, sowie die Schaltkontaktflächen 33c samt den zugehörigen Prüftasten und diverse elektrische Leitungen, in dem wieder verwendbaren Halteelement 1 angeordnet sind und nicht mehr - wie beim Stand der Technik - im nur einmal verwendbaren Druckmeßwandler.

Fig. 3 zeigt ein Ausführungsbeispiel einer Druckmeßwandleranordnung, bei der der Druckmeßwandler 12 vom Halteelement 1 abgenommen ist. Die beiden Anschlüsse 21 und 22 sind Enden eines Druckmeßkanals 39, der Teil eines Gehäuses 43 des Druckmeßwandlers 12 ist. In seinem Mittelbereich weist der Druckmeßkanal 39 einen kurzen Stichkanal 40 auf, dessen Ende hier unmittelbar durch ein Druckmeßelement 41 verschlossen ist. Alternativ dazu kann auf das Druckmeßelement 41 ein Zwischenmedium aufgebracht sein, beispielsweise ein Haftgel, so daß ein im Kanal 39 enthaltenes Fluid das Druckmeßelement nicht unmittelbar benetzt.

Das Druckmeßelement 41 ist auf einem Substrat 42 fixiert, das im Gehäuse 43 angeordnet ist. Über Verbindungsleitungen, von denen hier lediglich die beiden Verbindungsleitungen 64 und 65 zu sehen sind, ist das Druckmeßelement 41 elektrisch mit Kontaktstiften 62 bzw. 63 verbunden. Die Kontaktstifte 62 und 63 und das Substrat 42 sind an einem Verschlußteil 43a befestigt, das in das Gehäuse 43 eingesetzt ist und dieses an seiner Außenseite 44 verschließt. Alternativ zu dem hier gezeigten Ausführungsbeispiel, bei dem die Kontaktstifte 62 und 63 bündig mit der Außenseite des Gehäuses 43 abschließen, können sie auch von der Außenseite 44 abstehen oder gehäuseeinwärts versetzt sein.

Das Halteelement 1 weist ein oberes Gehäuseteil 45 und ein unteres Gehäuseteil 46 auf. In einem Hohlraum 46a des unteren Gehäuseteils 46 ist eine Leiterbahnplatte oder Substrat 47 angeordnet, das an seiner Oberseite 48 mit Leiterbahnen versehen ist, von denen in der gezeigten Seitenansicht lediglich die beiden Leiterbahnen 30 und 31 zu sehen sind. Die Leiterbahnen 30 und 31 sind mit Adern des Kabels 25 verbunden, sei es durch direktes Anlöten oder über ein Verbindungsteil 49, das beispielsweise eine Buchse mit Stecker sein kann.

Das obere Gehäuseteil 45 ist mit dem unteren Gehäuseteil 46 verbunden, beispielsweise durch Kleben, Ultraschallschweißen, formschlüssiges Einrasten etc. Im oberen Gehäuseteil 45 sind zwei Ausnehmungen 50 und 51 vorgesehen, in denen Kontaktstifte 52 bzw. 53 angeordnet sind, die einen Absatz 54 bzw. 55 aufweisen. Der Kontaktstift 52 ist durch eine Feder 56 mit der auf der Oberseite 48 des Substrats 47 vorgesehenen Leiterbahn 31 elektrisch verbunden, wobei ein Ende der Feder 56 unmittelbar gegen die Leiterbahn 31 und das andere Ende der Feder 56 gegen den Absatz 54 drückt. In analoger Weise ist der Kontaktstift 53 mit der Leiterbahn 30 bzw. mit dem Kabel 25 elektrisch verbunden. Ist wie in Fig. 3 der Druckmeßwandler 12 abgenommen, so sind die Kontaktstifte 52 und 53 durch die Federn 56 in ihrer Extremstellung gedrückt, in der die Absätze 54 und 55 am "Grund" der beiden Ausnehmungen anliegen.

Die Kontaktstifte 52 und 53 sind in dem hier gezeigten Ausführungsbeispiel so lang, daß ihre Kontaktflächen 57 von einer Oberseite 58 des oberen Gehäuseteils 45 abstehen. Im Bereich der Oberseite 58 ist am Gehäuseoberteil 45 ein elastisches Dichtorgan 59 befestigt, das die Mantelseite des Kontaktstiftes 52 umschließt und das Halteelement 1 abdichtet.

Das Gehäuseoberteil 45 weist an seiner Oberseite 58 ein Fixierelement 60 mit einem Rastelement 61 auf. Wird der Druckmeßwandler 12 auf das Halteelement 1 aufgesetzt, so berühren sich die Kontakte 62 und 52 bzw. 63 und 53, wodurch das Druckmeßelement 41 elektrisch mit dem Kabel 25 verbunden ist. Durch Niederdrücken des Druckmeßwandlers 12 werden die Kontaktstifte 52 und 53 entgegen den Federkräften so weit in die Halteelement 1 eingedrückt, bis das Gehäuse 43 des Druckmeßwandlers 12 in die Fixiereinrichtung 60 bzw. 61 einrastet. Hierbei verformen sich die elastischen Dichtorgane 59 und pressen eine ggf. auf der Oberseite 58 des Gehäuses 1 verbliebene Flüssigkeit zur Seite, wodurch ein eventueller Flüssigkeitsfilm unterbrochen wird.

Zusätzlich ist es möglich, die Kontakte 62 und 63 ungebrauchter Druckmeßwandler 12 mit einem Schutzfilm oder ähnlichem vor Feuchtigkeit bzw. Nässe zu schützen; der Schutz kann unmittelbar vor Gebrauch des Druckmeßwandlers 12 entfernt werden oder so angebracht sein, daß er durch das Ansetzen an das Halteelement 1 zerstört wird, indem beispielsweise die Kontakte 52 und 53 den Schutzfilm durchstechen.

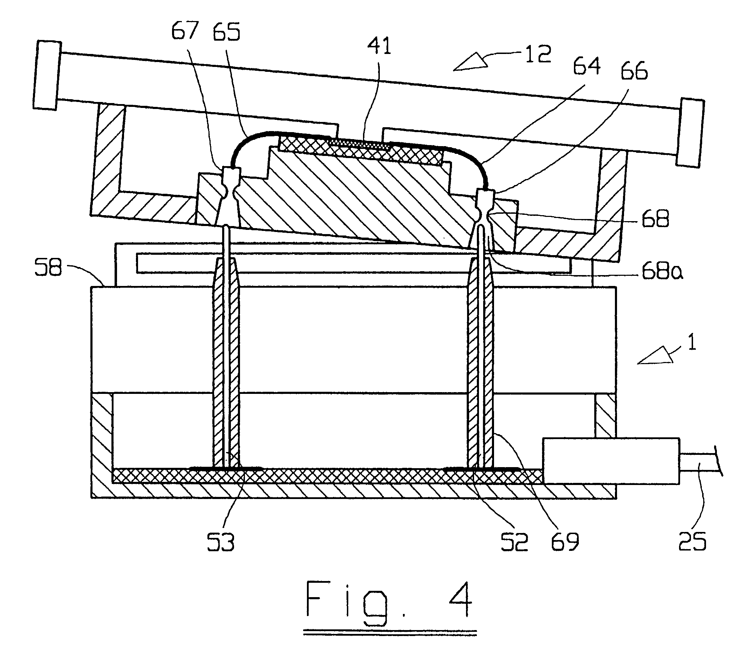

Fig. 4 zeigt ein Ausführungsbeispiel ähnlich dem der Fig. 3, wobei hier im Druckmeßwandler 12 Klemmkontakte 66 und 67 vorgesehen sind, die über die Leitungen 64 und 65 mit dem Druckmeßelement 41 verbunden sind. Die Klemmkontakte 66 und 67 weisen jeweils einen Klemmbuckel 68 auf und sind in einem Einführbereich 68a konisch gestaltet. Das Halteelement 1 weist Kontaktstifte 52 und 53 auf, die hier fest mit dem Halteteil 1 verbunden sind und die von dessen Oberseite 58 abstehen. Die Kontaktstifte 52 und 53 sind durch je ein zylindrisches Dichtorgan 69 aus elastischem Material umschlossen, das geringfügig von der Gehäuseoberseite 58 absteht und das dort konisch gestaltet ist, entsprechend den Einführbereichen 68a der Klemmkontakte 66 bzw. 67.

Durch Aufsetzen des Druckmeßwandlers 12 auf das Halteelement 1 dringen die Kontaktstifte 52 und 53 in die Klemmkontakte 66 und 67 ein, berühren die Klemmbuckel 68 und stellen so eine elektrische Verbindung zwischen dem Druckmeßelement 41 und dem Kabel 25 her. Hierbei verformen sich die Dichtorgane 69 geringfügig und dichten das Halteelement 1 ab, wobei ggf. auf den Kontaktstiften 53, 54 vorhandene Flüssigkeit abgestreift wird.

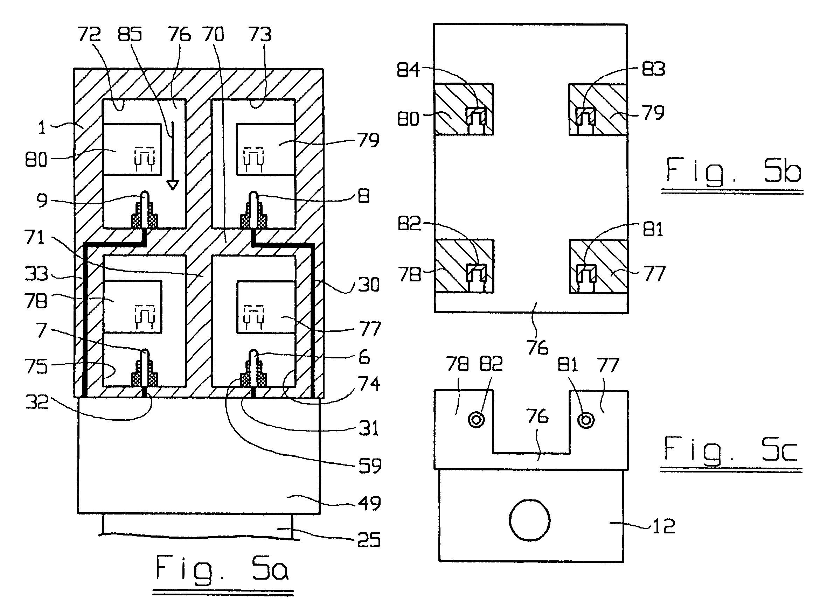

Die Fig. 5a, 5b und 5c zeigen ein weiteres Ausführungsbeispiel einer Druckmeßwandler-Anordnung, bei der die Halteplattenkontakte 6, 7, 8 und 9 ebenfalls Kontaktstifte sind, die aber im Unterschied zu den vorangegangenen Ausführungsbeispielen gegenüber der Außenmantelfläche in das Innere des Halteelements 1 versetzt ist. Das Halteelement 1 ist als Rahmen ausgebildet, der symmetrisch angeordnete Mittelstege 70 und 71 aufweist, wodurch vier rechteckförmige Durchgangsöffnungen 72, 73, 74 und 75 gebildet werden, in welche die Halteplattenkontakte 6, 7, 8 und 9 ragen. Die Halteplattenkontakte 6, 7, 8 und 9 sind über die elektrischen Leitungen 30, 31, 32 und 33 mit dem Verbindungsteil 49 und dem daran angeschlossenen Kabel 25 verbunden.

Der in Fig. 5c schematisch dargestellte Druckmeßwandler 12 ist mit einem plattenartigen Teil 76 verbunden, von dem senkrecht quaderförmige Blöcke 77, 78, 79 und 80 abstehen. In den Blöcken 77, 78, 79 und 80 sind Kontaktbuchsen 81, 82, 83 und 84 angeordnet, die elektrisch mit dem Druckmeßwandler 12 verbunden sind. Wird die Grundplatte 76 des Druckmeßwandlers 12 an das Halteelement 1 angesetzt, so ragen die Blöcke 77, 78, 79 und 80 in die Durchgangsöffnungen 72, 73, 74 und 75 (Fig. 5a). Durch Aufschieben des Druckmeßwandlers 12 in Pfeilrichtung 85 wird eine elektrische Verbindung zwischen den Kontaktbuchsen 81, 82, 83 und 84 des Druckmeßwandlers 12 und den Kontaktstiften 6, 7, 8 und 9 des Halteelements 1 hergestellt, wobei die an den Kontaktstiften 6, 7, 8 und 9 vorgesehenen elastischen Dichtorgane 59 verformt werden.

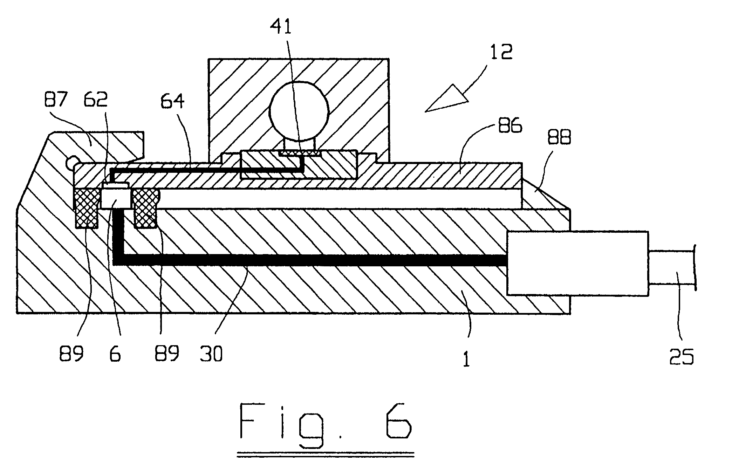

Fig. 6 zeigt ein Ausführungsbeispiel, bei dem der Druckmeßwandler 12 einen plattenartigen Abschnitt 86 aufweist, der zwischen einer Haltenase 87 und einem Rastbuckel 88 des Halteelements 1 eingerastet ist. Das Druckmeßelement 41 ist über elektrische Verbindungsleitungen, von denen hier nur die Verbindungsleitung 64 zu sehen ist, mit dem hier scheibenförmigen Kontakt 62 verbunden. Der Kontakt 62 berührt den Gegenkontakt 6, der außen am Halteelement 1 fixiert und mit dem Kabel 25 elektrisch verbunden ist. Der Gegenkontakt 6 ist von einem am Halteelement 1 fixierten elastischen Dichtorgan 89 umschlossen, das bei Ansetzen des plattenförmigen Abschnitts 86 des Druckmeßwandlers 12 an die Haltenase 87 ggf. am Abschnitt 86 verbliebene Flüssigkeitstropfen abwischt und den Kontaktbereich vor Feuchtigkeit schützt und zusätzlich zur Fixierung des Druckmeßwandlers 12 beiträgt.

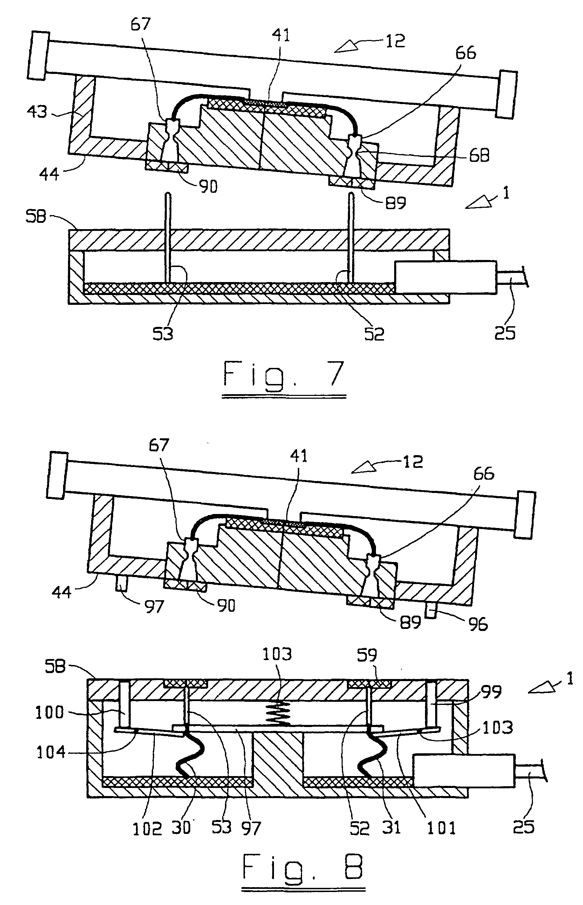

Fig. 7 zeigt ein Ausführungsbeispiel, bei dem die zur Außenseite 44 des Gehäuses 43 des Druckmeßwandlers 41 geführten Klemmkontakte 66 und 67 durch elastische Dichtorgane 89 und 90 abgedeckt und vor Feuchtigkeit geschützt sind. Bei Aufsetzen des Druckmeßwandlers 12 auf das Halteelement 1 durchdringen die fest mit dem Halteelement 1 verbundenen Halteplattenkontakte 52 und 53 die Dichtungen 89 und 90, berühren die zugeordneten Kontakte 62 und 63 und stellen so eine elektrische Verbindung zwischen dem Druckmeßelement 41 und dem Kabel 25 her.

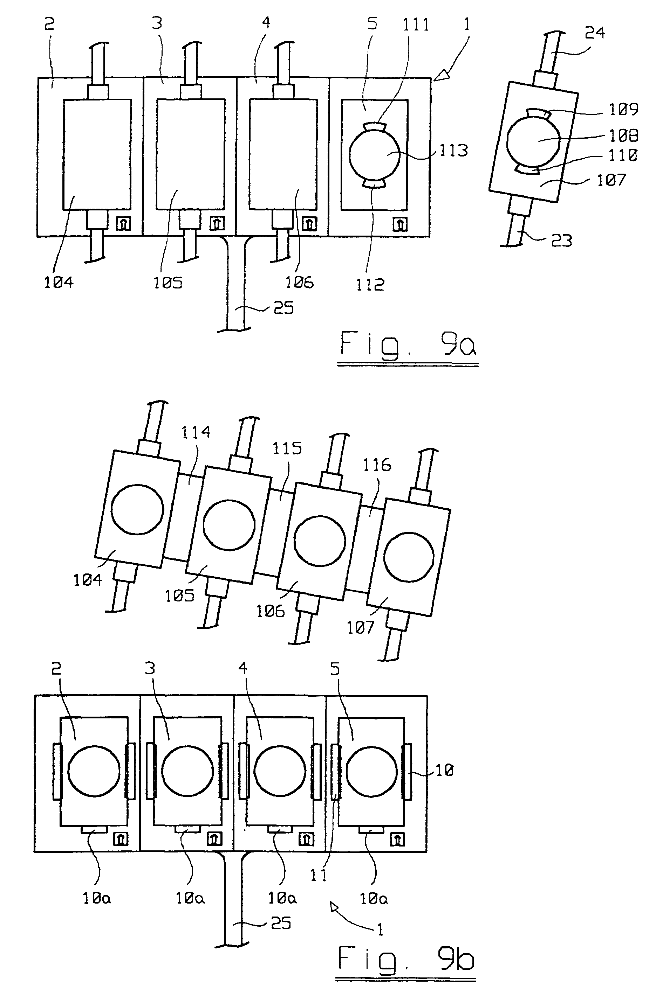

Fig. 8 zeigt ein Ausführungsbeispiel mit einem Druckmeßwandler 12, ähnlich dem der Fig. 8, wobei an der Unterseite 44 des Druckmeßwandlers 12 Erhebungen 96 und 97 vorgesehen sind. Die Halteplattenkontakte 52 und 53 sind bei diesem Ausführungsbeispiel auf einer Platte 97 fixiert, die in Längsrichtung der Kontakte 52 und 53 beweglich ist. Eine elektrische Verbindung zwischen dem Kabel 25 und den Kontakten 52 und 53 ist durch Verbindungsleitungen 30 bzw. 31 hergestellt, die so lang sind, daß ein ungehindertes Verschieben der Platte 97 möglich ist.

Alternativ dazu können die Kontakte 52 bzw. 53 auch über zugeordnete Federn, entsprechend Fig. 3, elektrisch mit dem Kabel 25 verbunden sein.

Im Halteelement 1 sind Druckstifte 99 und 100 vorgesehen, die auf Hebel 101 bzw. 102 drücken. Die Hebel 101 und 102 sind durch hier lediglich schematisch dargestellte Lager 103 bzw. 104 schwenkbar im Halteelement 1 gelagert. Wird der Druckmeßwandler 12 auf die Oberseite 58 des Halteelements 1 aufgesetzt, so drücken die Erhebungen 96 und 97 die Druckstifte 99 und 100 in das Halteelement (1), wodurch die Hebel 101 und 102 verschwenkt werden und die Platte 97 mit den daran fixierten Halteplattenkontakten 52 und 53 entgegen der Kraft einer Rückstellfeder 103 in die Kontakte 62 und 63 des Druckmeßwandlers 12 eingeschoben werden.

Fig. 9a zeigt ein Ausführungsbeispiel, bei dem einmal verwendbare Druckdome 104-107 vorgesehen sind, die in ihrem Inneren einen Hohlraum aufweisen, der über den Schlauch 23 mit dem Gefäßsystem des Patienten in Druckverbindung steht und über den Schlauch 24 an die Infusionsflasche (nicht dargestellt) angeschlossen ist. An einer Außenseite der Druckdome 104-107 ist jeweils eine elastische Druckübertragungsmembran 108 vorgesehen, die ebenfalls mit dem Hohlraum in Druckverbindung steht. Die Druckdome 104-107 weisen im Bereich der Druckübertragungsmembran 108 Fixierelemente 109 bzw. 110 auf, die mit Fixierelementen 111 bzw. 112 zusammenwirken, die an den Druckmeßplätzen 2-5 vorgesehen sind. Die Fixierelemente 109-112 sind hier als Bajonettverschlußelemente ausgeführt. Zum Befestigen der Druckdome 104-107 werden diese "schräg" auf die Druckmeßplätze 2-5 aufgesetzt und durch seitliches Drehen fixiert, wobei die Fixierelemente 109-112 ineinandergreifen. Alternativ können auch Rastelemente entsprechend den Rastelementen 10 und 11 der Fig. 1 vorgesehen sein, so daß die Druckdome geradlinig auf das Halteelement 1 aufgeschoben werden können. Durch Anschläge 10a wird die Endlage der Druckdome eindeutig definiert.

Die Druckmeßplätze 2-5 weisen ebenfalls je eine Druckübertragungsmembran 113 auf, die bei aufgesetztem Druckdom 104-107 die zugeordnete Druckübertragungsmembran 108 berührt. Die an den einzelnen Druckmeßplätzen 2-5 vorgesehenen Druckübertragungsmembranen 113 stehen mit Druckmeßelementen (nicht dargestellt) in Druckverbindung, die hier in das wiederverwendbare Halteelement 1 integriert sind. Die in den Druckdomen 104-107 herrschenden Fluiddrücke werden über die Druckübertragungsmembranen 108 bzw. 113 auf die Druckmeßelemente übertragen und von diesen in elektrische Signale umgewandelt, die über das gemeinsame Kabel 25 abgreifbar sind.

Fig. 9b zeigt ein Ausführungsbeispiel, bei dem die Druckdome 104-107 durch Verbindungselemente 114-116 miteinander verbunden sind und als "eine Einheit" auf die Steckplätze 2-5 aufgesetzt werden, die hier - analog zum Ausführungsbeispiel der Fig. 1 - jeweils Rastelemente 10 bzw. 11 zur Fixierung der Druckdome 104-107 aufweisen.

Fig. 10a zeigt ein Ausführungsbeispiel, bei dem das an die Druckmeßplätze 2-5 angeschlossene Kabel 25 fest mit dem Halteelement 1 verbundenen ist. Am freien Ende des Kabels 25 ist hier ein Stecker 117 vorgesehen, der in eine Buchse 118 eingesteckt ist. Von der Buchse 118 führen einzelne Monitorkabel 119-122 weg, die den Druckmeßplätzen 2-5 zugeordnet sind und die über zugeordnete Monitorstecker 123-126 mit dem Monitor 20 verbindbar sind. Bei einer Beschädigung des Kabels 25 bzw. des Halteelements 1 können die Monitorkabel 119-122 und die Monitorstecker 123-126 wieder verwendet werden.

Bei Fig. 10b ist eine zusätzliche elektrische Steckverbindung 127 vorgesehen, wobei ein Verbindungselement 128 unmittelbar am Halteelement 1 angeordnet ist und über ein Verbindungselement 129 an das Kabel 25 angeschlossen ist. Im Vergleich zu Fig. 10a können hier die Halteplatte 1 und das Kabel 25 unabhängig voneinander ausgetauscht werden.

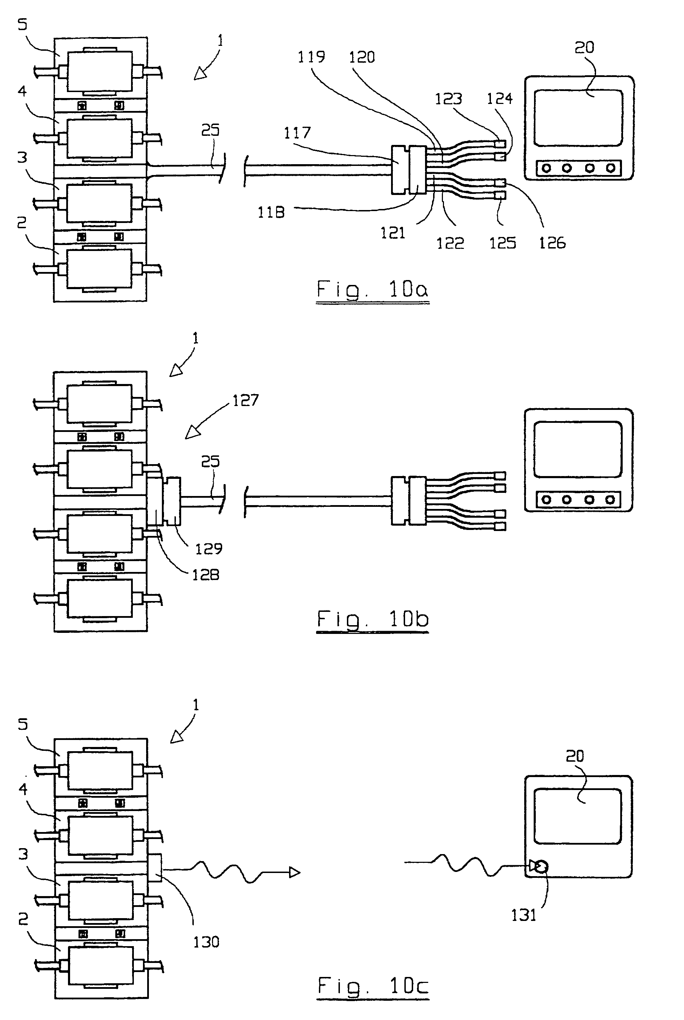

Fig. 10c zeigt ein Ausführungsbeispiel einer kabellosen Datenübertragung zwischen den Druckmeßelementen des Halteelements 1 und dem Monitor 20. Hierfür ist am Halteelement 1 eine Sendeeinrichtung 130 vorgesehen, die mit den Druckmeßelementen der Druckmeßplätze 2-5 verbunden ist und elektromagnetische Signale aussendet, die den gemessenen Drücken entsprechen. Diese Signale werden dann von einer Empfangseinheit 131 des Monitors 20 empfangen und angezeigt.

## Patentansprüche

1. Druckmeßwandler-Anordnung zur Messung des Druckes eines Fluids, insbesondere zur invasiven Blutdruckmessung, mit mehreren einmal verwendbaren Druckmesswandlern (12-15), die je ein Gehäuse aufweisen, in dem ein Druckmeßelement angeordnet ist, das mit dem zu messenden Druck beaufschlagbar ist und das über elektrische Verbindungsmittel mit elektrischen Kontakten verbunden ist,
mit einem Halteelement (1), an dem die Druckmeßwandler fixierbar sind, wobei am Halteelement (1) für jeden Druckmeßwandler (12-15) je eine Gruppe von Gegenkontakten (6-9, 52, 53) vorgesehen ist und sämtliche Gegenkontakte mit einer einzigen gemeinsamen Signalübertragungseinrichtung (25, 130) für alle Druckmeßwandler (12-15) verbunden sind,
**dadurch gekennzeichnet,**
**daß** die einzige gemeinsame Signalübertragungseinrichtung (25, 130) mittels einer von Hand lösbaren elektrischen Steckverbindung (128, 129) mit der Halteeinrichtung (1) verbunden ist, und
**daß** das dem Halteelement (1) abgewandte Ende (27) des Kabels (25) über eine Stecker-/Buchsenanordnung (28, 118) mit einer der Anzahl der Druckmeßwandler (12-15) entsprechenden Anzahl von Kabeln (119, 120, 121, 122) verbunden ist, an deren freien Enden Monitorstecker (123, 124, 125, 126) angebracht sind, wobei diese Kabel (119-122) kürzer sind als das gemeinsame Kabel (25), das unmittelbar an die Halteeinrichtung angeschlossen ist.

2. Druckmeßwandler-Anordnung nach Anspruch 1, **dadurch gekennzeichnet,**
**daß** sämtliche Gegenkontakte (6-9) am Halteelement (1) an der Außenfläche des Halteelementes (1) angeordnet sind.

3. Druckmeßwandler-Anordnung nach Anspruch 2, **dadurch gekennzeichnet,**
**daß** die Gegenkontakte (6-9, 52, 53) von der Außenfläche (58) des Halteelementes (1) abstehen.

4. Druckmeßwandler-Anordnung nach Anspruch 3, **dadurch gekennzeichnet,**
**daß** die Gegenkontakte Kontaktstifte (6-9, 52, 53) sind.

5. Druckmeßwandler-Anordnung nach einem der Ansprüche 1 bis 4, **dadurch gekennzeichnet,**
**daß** das Halteelement (1) einen Hohlraum (50, 51, 46a) aufweist, in den die Gegenkontakte (52, 53) ragen,
**daß** im Innern des Halteelementes (1) elektrische Leitungsmittel (30, 31) angeordnet sind, die mit jeweils einer Ader (35, 36, 37, 38) des einzigen Kabels (25) verbunden sind und
**daß** jeder Gegenkontakt (52, 53) über eine zugeordnete Feder (56) jeweils mit einem der Leitungsmittel (30, 31) elektrisch verbunden ist.

6. Druckmeßwandler-Anordnung nach einem der Ansprüche 1 bis 5, **dadurch gekennzeichnet,**
**daß** die Gegenkontakte (6, 7, 8, 9) Kontaktstifte sind, die so am Halteelement (1) angeordnet sind, daß sie im wesentlichen parallel zu einer durch die Oberfläche (58) des Halteelementes (1) gebildeten Ebene sind.

7. Druckmeßwandler-Anordnung nach Anspruch 6, **dadurch gekennzeichnet,**
**daß** das Halteelement (1) ein Rahmen mit mindestens einem Quersteg (70, 71) ist, wobei durch den mindestens einen Quersteg (70, 71) Rahmenparzellen (72, 73, 74, 75) gebildet sind,
**daß** ein Teil der Gegenkontakte (8, 9) an dem Quersteg (70, 71) und die anderen Gegenkontakte (6, 7) am Rahmen fixiert sind,
**daß** der Druckmeßwandler (12, 13, 14, 15) ein plattenartiges Teil (76) mit abstehenden Blöcken (77, 78, 79, 80) aufweist, in denen die Kontakte (81, 82, 83, 84) angeordnet sind, wobei die Blöcke (77, 78, 79, 80) so dimensioniert sind, daß sie in die Rahmenparzellen (72, 73, 74, 75) einführbar sind.

8. Druckmeßwandler-Anordnung nach einem der Ansprüche 1 bis 6, **dadurch gekennzeichnet,**
**daß** die Kontakte (66, 67; 81-84) oder die Gegenkontakte (6, 7, 8, 9) Klemmkontakte sind.

9. Druckmeßwandler-Anordnung nach einem der Ansprüche 1 bis 8, **dadurch gekennzeichnet,**
**daß** am Halteelement (1) elastische Dichtorgane (59, 69, 89) vorgesehen sind, welche die Gegenkontakte (6-9; 52, 53) im Bereich der Außenseite (58) des Halteelements (1) teilweise umschließen.

10. Druckmeßwandleranordnung nach einem der Ansprüche 3 bis 9, **dadurch gekennzeichnet,**
**daß** mehrere Druckmeßwandler (12-15) fest miteinander verbunden sind und gemeinsam auf der Halteplatte (1) fixierbar sind.

11. Druckmeßwandler-Anordnung zur Druckmessung von Fluiden, insbesondere zur invasiven Blutdruckmessungmit einem Druckdom, der ein Gehäuse mit einem Innenraum zur Aufnahme des Fluids und einer den Innenraum begrenzenden flexiblen Druckübertragungsmembran besteht, mit einem Halteelement, an dem der Druckdom von Hand fixierbar ist, wobei das Halteelement eine zweite Druckübertragungsmembran aufweist, die mit der Druckübertragungsmembran des Druckdomes in Berührung steht, mit einem Druckmeßelement, das dem Fluiddruck entsprechende elektrische Signale erzeugt und mit einer mit dem Halteelement verbundenen Signalübertragungseinrichtung zur Übertragung der elektrischen Signale zu einem Aufzeichnungs-/Anzeigegerät,
**dadurch gekennzeichnet,**
**daß** am Halteelement (1) mehrere Druckdome (104-107) fixierbar sind,
**daß** am Halteelement (1) für jeden Druckdom (104-107) je eine Druckübertragungsmembran (113) vorgesehen ist, die jeweils mit je einem Druckmeßwandler gekoppelt ist,
**daß** alle Druckmeßwandler mit einer einzigen gemeinsamen Signalübertragungseinrichtung (125, 130) für alle Druckmeßwandler verbunden sind,
**daß** die einzige gemeinsame Signalübertragungseinrichtung (25, 130) mittels einer von Hand lösbaren elektrischen Steckverbindung (128, 129) mit der Halteeinrichtung (1) verbunden ist, und
**daß** das dem Halteelement (1) abgewandte Ende (27) des Kabels (25) über eine Stecker-/Buchsenanordnung (28, 118) mit einer der Anzahl der Druckmeßwandler (12-15) entsprechenden Anzahl von Kabeln (119, 120, 121, 122) verbunden ist, an deren freien Enden Monitorstecker (123, 124, 125, 126) angebracht sind, wobei diese Kabel (119-122) kürzer sind als das gemeinsame Kabel (25), das unmittelbar an die Halteeinrichtung angeschlossen ist.

12. Druckmeßwandleranordnung nach Anspruch 11, **dadurch gekennzeichnet,**
**daß** mehrere Druckdome (104-107) zu einer Einheit miteinander verbunden sind und gemeinsam am Halteelement (1) fixierbar sind.

## Claims

1. Pressure transducer arrangement for the measuring of fluid pressure, particularly for invasive blood pressure measuring, with several disposable pressure transducers (12-15), each having a housing containing a pressure-measuring element that can be charged with the pressure to be measured and that is connected to electric contacts by means of electric connecting devices,
with a holding element (1) to which the pressure transducer can be attached, wherein on the holding element (1) for each pressure transducer (12-15) each a group of counter-contacts (6-9, 52, 53) is provided and all counter-contacts are connected with a single common signal transmitting device (25, 130) for all pressure transducers (12-15),
**characterized in that**
the single common signal transmitting device (25, 130) is attached to the holding device (1) by means of an electric plug-and-socket connection (128, 129) which is detachable by hand, and
that the end (27) of cable (25) that is pointing away from the holding element (1) is connected via a plug-and-socket-arrangement to a number of cables (119, 120, 121, 122) that is equal to the number of pressure transducers (12-15), the free ends of cables bear monitor plugs (123, 124, 125, 126), wherein these cables (119-122) are shorter than the common cable (25) that is directly connected to the holding device.

2. Pressure transducer according to Claim 1, **characterized by** the fact
that all counter-contacts (6-9) of the holding element (1) are located on the external surface of holding element (1).

3. Pressure transducer according to Claim 2, **characterized by** the fact
that the counter-contacts (6-9, 52, 53) protrude above the external surface (58) of the holding element (1).

4. Pressure transducer arrangement according to Claim 3, **characterized by** the fact
that the counter-contacts are contact pins (6-9, 52, 53).

5. Pressure transducer arrangement according to one of Claims 1 to 4, **characterized by** the fact
that the holding element (1) features a cavity (50, 51, 46a) into which the counter-contacts (52, 53) extend,
that inside the holding element (1) electric lines (30, 31) are configured that are each connected to a wire (35, 36, 37, 38) of said single cable (25) and
that each counter-contact (52, 53) is electrically connected to a line (30, 31) via a corresponding spring (56).

6. Pressure transducer arrangement according to one of Claims 1 to 5, **characterized by** the fact
that the counter-contacts (6, 7, 8, 9) are contact pins that are arranged on the holding element (1) in such a way that they are essentially parallel with the level of a surface (58) of the holding element (1).

7. Pressure transducer arrangement according to Claim 6, **characterized by** the fact
that the holding element (1) is a frame with at least one crosspiece (70, 71), wherein by means of at least one of the aforementioned crosspieces (70, 71) frame cells (72, 73, 74, 75) are formed,
that some of the counter contacts (8, 9) are attached to the crosspiece (70, 71), wherein the other counter-contacts (6, 7) are attached to the frame,
that the pressure transducer (12, 13, 14, 15) features a plate-shaped part (76) with protruding blocks (77, 78, 79, 80) in which contacts (81, 82, 83, 84) are located, whereas blocks (77, 78, 79, 80) are dimensioned in such a way as to be introducible into the frame cells (72, 73, 74, 75).

8. Pressure transducer arrangement according to one of Claims 1 to 6, **characterized by** the fact
that the contacts (66, 67; 81-84) or the counter-contacts (6, 7, 8, 9) are clamping contacts.

9. Pressure transducer arrangement according to one of Claims 1 to 8, **characterized by** the fact
that the holding element (1) features elastic sealing elements (59, 69, 89) that partially envelop the counter-contacts (6-9, 52, 53) in the area of the external surface (58) of holding element (1).

10. Pressure transducer arrangement according to one of Claims 3 to 9, **characterized by** the fact
that several pressure transducers (12-15) are firmly attached to each other and are together mounted on holding plate (1).

11. Pressure transducer arrangement for the measurement of pressure of fluids, particularly for invasive measuring of blood pressure with a pressure dome consisting of a housing with an internal cavity for the reception of the fluid and of a flexible pressure transfer membrane that defines the interior space, with a holding element to which the pressure dome can be manually attached, wherein the holding element features a second pressure transfer membrane that touches the pressure transfer membrane of the pressure dome, with a pressure measuring element that produces electric signals that correspond to the fluid pressure and with a signal transfer device connected to the holding element for the transfer of the electric signals to a display/recording device,
**characterized by** the fact
that several pressure domes (104-107) can be attached to the holding element (1),
that a respective transfer membrane (113) is provided at the holding element (1) for each pressure dome (104-107) and each membrane is coupled to an individual pressure transducer,
that all pressure transducers are connected to a common signal transfer line (125, 130) that serves all of the pressure transducers,
that the single common signal transmitting device (25, 130) is attached to the holding device (1) by means of an electric plug-and-socket connection (128, 129) which is detachable by hand, and
that the end (27) of cable (25) that is pointing away from the holding element (1) is connected via a plug-and-socket-arrangement to a number of cables (119, 120, 121, 122) that is equal to the number of pressure transducers (12-15), the free ends of cables bear monitor plugs (123, 124, 125, 126), wherein these cables (119-122) are shorter than the common cable (25) that is directly connected to the holding device.

12. Pressure transducer arrangement according to Claim 11, **characterized by** the fact
that several pressure domes (104-107) are combined with each other into one unit and are together attached to holding element (1).

## Revendications

1. Dispositif de mesure de pression pour la mesure de la pression d'un fluide, notamment pour la mesure invasive de la pression sanguine, avec plusieurs transducteurs de pression (12-15) utilisables une fois, qui présentent chacun un corps, dans lequel est monté un élément de mesure de pression qui peut être soumis à la pression à mesurer et qui est relié à des contacts électriques par des liaisons électriques,
avec un élément de support (1) sur lequel les transducteurs de pression peuvent être fixés, l'élément de support (1) présentant pour chaque transducteur de pression (12-15) un groupe de contre-contacts respectif (6-9, 52, 53) et tous les contre-contacts étant reliés à un unique système de transmission de signaux (25, 130) commun pour tous les transducteurs de pression (12-15),
**caractérisé en ce que**
l'unique système de transmission de signaux commun (25, 130) est relié au système de support (1) à l'aide d'un connecteur électrique (128, 129) détachable à la main, et
l'extrémité (27) du câble (25) opposée à l'élément de support (1) est reliée par un dispositif fiche/prise (28, 118) à un nombre de câbles (119, 120, 121, 122) correspondant au nombre de transducteurs de pression (12-15), des fiches de moniteur (123, 124, 125, 126) étant montées sur les extrémités libres desdits câbles (119-122), lesquels sont plus courts que le câble commun (25) directement raccordé au système de support.

2. Dispositif de mesure de pression selon la revendication 1,
**caractérisé en ce que**
tous les contre-contacts (6-9) de l'élément de support (1) sont montés sur la face extérieure de l'élément de support (1).

3. Dispositif de mesure de pression selon la revendication 2,
**caractérisé en ce que**
les contre-contacts (6-9, 52, 53) saillent de la face extérieure (58) de l'élément de support (1).

4. Dispositif de mesure de pression selon la revendication 3,
**caractérisé en ce que**
les contre-contacts sont des fiches de contact (6-9, 52, 53).

5. Dispositif de mesure de pression selon l'une des revendications 1 à 4, **caractérisé en ce que**
l'élément de support (1) présente une cavité (50, 51, 46a) dans laquelle dépassent les contre-contacts (52, 53), et à l'intérieur de l'élément de support (1) sont montés des fils électriques (30, 31) qui sont reliés respectivement à un brin (35, 36, 37, 38) du câble unique (25) et
chaque contre-contact (52, 53) est respectivement relié électriquement à l'un des fils (30, 31) par un ressort correspondant (56).

6. Dispositif de mesure de pression selon l'une des revendications 1 à 5, **caractérisé en ce que**
les contre-contacts (6, 7, 8, 9) sont des fiches de contact qui sont disposées sur l'élément de support (1) de telle sorte qu'elles sont essentiellement parallèles à un plan formé par la surface (58) de l'élément de support (1).

7. Dispositif de mesure de pression selon la revendication 6, **caractérisé en ce que**
l'élément de support (1) est un cadre avec au moins une entretoise (70, 71), des parcelles de cadre (72, 73, 74, 75) étant formées par ladite au moins une entretoise (70, 71),
une partie des contre-contacts (8, 9) est fixée à l'entretoise (70, 71), tandis que les autres contre-contacts (6, 7) sont fixés au cadre,
le transducteur de pression (12, 13, 14, 15) présente une pièce en forme de plaque (76) avec des blocs saillants (77, 78, 79, 80), dans lesquels sont disposés les contacts (81, 82, 83, 84), les blocs (77, 78, 79, 80) étant dimensionnés de telle sorte qu'ils sont insérables dans les parcelles de cadre (72, 73, 74, 75).

8. Dispositif de mesure de pression selon l'une des revendications 1 à 6, **caractérisé en ce que**
les contacts (66, 67 ; 81-84) ou les contre-contacts (6, 7, 8, 9) sont des contacts à bornes.

9. Dispositif de mesure de pression selon l'une des revendications 1 à 8, **caractérisé en ce que**
l'on prévoit sur l'élément de support (1) des organes d'étanchéité élastiques (59, 69, 89), qui entourent partiellement les contre-contacts (6-9; 52, 53) dans la zone de la face externe (58) de l'élément de support (1).

10. Dispositif de mesure de pression selon l'une des revendications 3 à 9, **caractérisé en ce que**
plusieurs transducteurs de pression (12-15) sont reliés entre eux de façon fixe et peuvent être fixés ensemble sur l'élément de support (1).

11. Dispositif de mesure de pression pour la mesure de pression des fluides, notamment pour la mesure invasive de la pression sanguine avec un dôme de pression, lequel est composé d'un corps dont l'intérieur est destiné à recevoir le fluide et d'une membrane de transmission de pression flexible délimitant l'intérieur, avec un élément de support sur lequel le dôme de pression peut être fixé manuellement, l'élément de support présentant une deuxième membrane de transmission de pression qui est en contact avec la membrane de transmission de pression du dôme de pression, avec un élément de mesure de pression qui génère des signaux électriques correspondant à la pression du fluide, et avec un système de transmission de signaux relié à l'élément de support et destiné à la transmission des signaux électriques vers un appareil d'enregistrement/d'affichage,
**caractérisé en ce que**
plusieurs dômes de pression (104-107) peuvent être fixés sur l'élément de support (1),
on prévoit sur l'élément de support (1) respectivement pour chaque dôme de pression (104-107) une membrane de transmission de pression (113), qui est couplée à un transducteur de pression respectif,
tous les transducteurs de pression sont reliés à un unique système de transmission de signaux (125, 130) commun pour tous les transducteurs de pression,
l'unique système de transmission de signaux commun (25, 130) est relié à l'élément de support (1) par un connecteur électrique (128, 129) détachable à la main, et
l'extrémité (27) du câble (25) opposée à l'élément de support (1) est reliée par un dispositif fiche/prise (28, 118) à un nombre de câbles (119, 120, 121, 122) correspondant au nombre de transducteurs de pression (12-15), des fiches de moniteur (123, 124, 125, 126) étant montées sur les extrémités libres desdits câbles (119-122), lesquels sont plus courts que le câble commun (25) directement raccordé au système de support.

12. Dispositif de mesure de pression selon la revendication 11,
**caractérisé en ce que**
plusieurs dômes de pression (104-107) sont reliés entre eux pour former une unité et peuvent être fixés ensemble sur l'élément de support (1).
